(19)

Europäisches
Patentamt
European
Patent Office
Office européen
des brevets

(11) **EP 2 568 284 A2**

(12) **EUROPEAN PATENT APPLICATION**

(43) Date of publication:
**13.03.2013  Bulletin 2013/11**

(51) Int Cl.:
***G01N 33/44*** *(2006.01)*

(21) Application number: **12005295.6**

(22) Date of filing: **19.07.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **09.09.2011  JP 2011197464**

(71) Applicant: **Sumitomo Rubber Industries, Ltd.
Hyogo-ken (JP)**

(72) Inventors:
• **Naito, Masato
  Kobe-shi
  Hyogo 651-0072 (JP)**
• **Ito, Wakana
  Kobe-shi
  Hyogo 651-0072 (JP)**

(74) Representative: **Manitz, Finsterwald & Partner
GbR
Postfach 31 02 20
80102 München (DE)**

(54)  **Method for simulating deformation of rubber compound with filler particles**

(57)   A method for simulating deformation of rubber compound with filler particles, comprises the following steps: by the use of a scanning transmission electron microscope (STEM), data of STEM images of the rubber compound are acquired; based on the data of the STEM images, a dataset of a three-dimensional structure of the rubber compound is reconstructed; based on the dataset of the three-dimensional structure of the rubber compound, a finite element model of the rubber compound is generated so that the model comprises a domain of a rubber component divided into a finite number of elements, and domains of the filler particles each divided into a finite number of elements; on the elements of the rubber component, the stress dependence on strain rate of the rubber component is defined; and based on the finite element model, a simulation of deformation of the rubber compound is carried out.

**FIG.2**

start

using scanning transmission
electron microscope, STEM
image data of rubber compound
are acquired — S1

based on STEM image data,
dataset of 3D structure of rubber
compound is reconstructed — S2

based on dataset of 3D structure,
slice image data of rubber
compound are reconstructed — S3

based on slice image data, finite
element model of rubber
compound is generated — S4

using finite element
model, deformation
simulation is carried out — S5

end

EP 2 568 284 A2

**Description**

**Background of the invention**

The present invention relates to a computerized method for simulating deformation of rubber compound with filler particles, more particularly to a method for generating a finite element model of the rubber compound capable of accurately simulating the deformation.

In general, rubber compounds used in rubber products, e.g. tires and the like contain fillers, e.g. carbon black, silica and the like as reinforcing agents.

It is generally known in the art that a dispersion of such filler particles in a rubber compound affects characteristics such as strength of the rubber compound. But, the full details of the relationships between the characteristics of a rubber compound and filler particles therein are not yet clear. Therefore, when making simulations or analyses of a rubber compound, it is important to use of an accurate finite element model of a rubber compound in which the dispersion of filler particles in the real rubber compound is accurately duplicated.

On the other hand, a rubber compound generally shows different viscoelastic properties depending on the strain rate. Accordingly, it is also important for precise simulations to generate a finite element model having the stress dependence on strain rate of the rubber compound.

**Summary of the Invention**

It is therefor an object of the present invention to provide a method for simulating deformation of rubber compound with filler particles in which a finite element model of the rubber compound in which a dispersion of the filler particles and the stress dependence on strain rate are accurately simulated, can be generated and thereby it is possible to make a precise simulation of deformation of the rubber compound and analyses of the rubber compound.

According to the present invention, a method for simulating deformation of rubber compound including a rubber component and filler particles, comprises:

a STEM image acquiring step in which, by the use of a scanning transmission electron microscope (STEM), data of STEM images of the rubber compound are acquired;

a three-dimensional structure reconstruction step in which, based on the data of the STEM images, a dataset of a three-dimensional structure of the rubber compound is reconstructed;

a finite element model generating step in which, based on the dataset of the three-dimensional structure of the rubber compound, a finite element model of the rubber compound is generated, so that the finite element model comprises a domain of the rubber component divided into a finite number of elements, and domains of the filler particles each divided into a finite number of elements;

a stress dependence defining step in which the stress dependence on strain rate of the rubber component is defined on the elements of the rubber component; and

a simulation step in which, based on the finite element model, a simulation of deformation of the rubber compound is carried out.

In the STEM image acquiring step, the focal point of the scanning transmission electron microscope is preferably set in a thickness center region of a specimen of the rubber compound. In the STEM image acquiring step, it is preferable that a specimen of the rubber compound is tilted with respect to the central axis of the scanning transmission electron microscope, and the STEM images are took at different tilt angles of the specimen of the rubber compound, while the focal point of the scanning transmission electron microscope is set in a thickness center region of the specimen of the rubber compound based on an apparent thickness measured along the direction of the electron beam axis across the specimen of the rubber compound. The thickness of the specimen of the rubber compound is preferably 200 to 1500 nm. The distance between the specimen of the rubber compound and a detector for the transmission electrons of the scanning transmission electron microscope is preferably 8 to 150 cm.

In the present invention, therefore, a finite element model resembling closely to the real rubber compound can be obtained. Accordingly, by making deformation calculations based on such finite element model, accurate simulation results can be obtained.

Further, as the stress dependence on strain rate is defined, simulation results useful for developing the rubber compound can be obtained. For instance, actual measurements of viscous elasticity of a rubber compound becomes very difficult if the frequency of the cyclic strain applied thereto exceeds 1000 Hz, whereas a frequency higher than 1000 Hz is required in order to assess grip performance of a tread rubber compound. In this invention, however, the assessment under cyclic strain of over 1000 Hz is possible.

By setting the focal point in a thickness center region of the specimen, the range on the specimen in which a clear image can be obtained becomes increased, and thereby it becomes possible to obtain the rubber compound model in which the dispersion of the filler particles is more accurately simulated.

Brief Description of the Drawings

Fig. 1 is a microscopical cross sectional view of a simplified example of a rubber compound.
Fig. 2 is a flow chart for explaining a method for simulating deformation of rubber compound as an embodiment of the present invention.
Fig. 3 is a diagram showing a scanning transmission electron microscope used in the method according to the present invention.
Fig. 4 is a diagram showing a scattering angle limiting aperture for dark-field image.
Fig. 5 is a diagram for explaining a device for tilting the specimen.
Fig. 6(a) is a schematic sectional view of the specimen for explaining the position of the focal point, taken along a section including the electron beam axis which is perpendicular to the incidence plane.
Fig. 6(b) is a schematic sectional view of the specimen for explaining the position of the focal point, taken along a section including the electron beam axis which inclines with respect to the incidence plane.
Fig. 7 is a schematic sectional view for explaining relationships among the depth of field of a scanning transmission electron microscope, a position of the focal point of the microscope, and the thickness of a specimen.
Fig. 8 is a perspective view produced from a dataset of the three-dimensional structure of a rubber compound.
Fig. 9(a) is a diagram showing a small part of a finite element model of a simplified example of a rubber compound.
Fig. 9(b) is a closeup thereof.
Fig. 10 is a perspective view of a three-dimensional finite element model of a simplified example of a rubber compound.
Fig. 11 shows slice images at an upper position and a lower position of a specimen of a rubber compound obtained under the after-mentioned different test conditions 1 and 2.
Fig. 12 is a cross sectional view of the specimen for explaining the upper position and lower position referred to in Fig. 11.
Fig. 13 is a graph showing stress-strain relationships as simulation results and actual measurements.

Description of the Preferred Embodiments

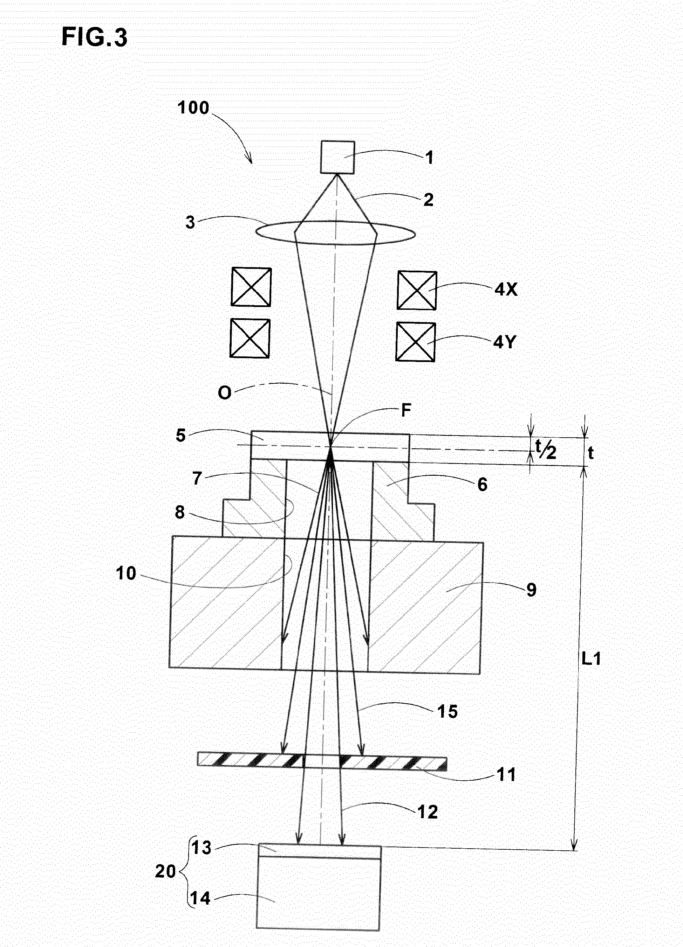

Embodiments of the present invention will now be described in detail in conjunction with accompanying drawings.
In the present invention, a two-dimensional or three-dimensional finite element model of the rubber compound (c) is generated by the use of a scanning transmission electron microscope 100 shown in Fig. 3 and a computer (not shown). Then, using the computer, a simulation of deformation of the rubber compound is carried out by making deformation calculation on the finite element model in order to analyze the rubber compound (c).

* Scanning transmission electron microscope

As usual, the scanning transmission electron microscope (STEM) 100 comprises: an electron gun 1 directed perpendicularly to a horizontal plane and capable of emitting electrons downward; a focusing lens 3 for focusing the electrons as an electron beam 2 on a specimen 5 of the rubber compound (c); scanning coils 4 including an X-direction scanning coil 4x and a Y-direction scanning coil 4Y for deflecting the electron beam 2 in the X-direction and Y-direction to scan the specimen 5; a specimen holder 6 for holding the specimen 5; and a specimen stage 9 on which the specimen holder 6 is detachably fixed. In a central portion of the specimen holder 6, an electron beam pass-through hole 8 is formed along the central axis (O) of the scanning transmission electron microscope 100 so that transmission electrons 7 which penetrate through the specimen 5 can pass through the hole 8.
In a central portion of the specimen stage 9, an electron beam pass-through hole 10 is formed along the central axis (O) and continuously from the electron beam pass-through hole 8 so that the transmission electrons 7 can pass through the hole 10. The microscope 100 further provided on the downstream side of the specimen stage 9 with a scattering angle limiting aperture 11 in order to limit the passing-through of the transmission electrons 7.
Further, on the downstream side of the scattering angle limiting aperture 11, there is disposed a detector 20 for the transmission electrons 15 passing through the aperture 11. The detector 20 comprises a scintillator 13 and a photoelectron multiplier tube 14.
The scintillator 13 reemits the energy of the incident electrons 12 passing though the aperture 11, in the form of light. The photoelectron multiplier tube 14 converts the incident light from the scintillator 13 to an electronic signal. Incidentally, the above-mentioned specimen stage 9, scattering angle limiting aperture 11, scintillator 13 and photoelectron multiplier tube 14 are arranged in a specimen room of a casing main body (not shown) of the microscope system 100.

* Rubber compound as analysis object

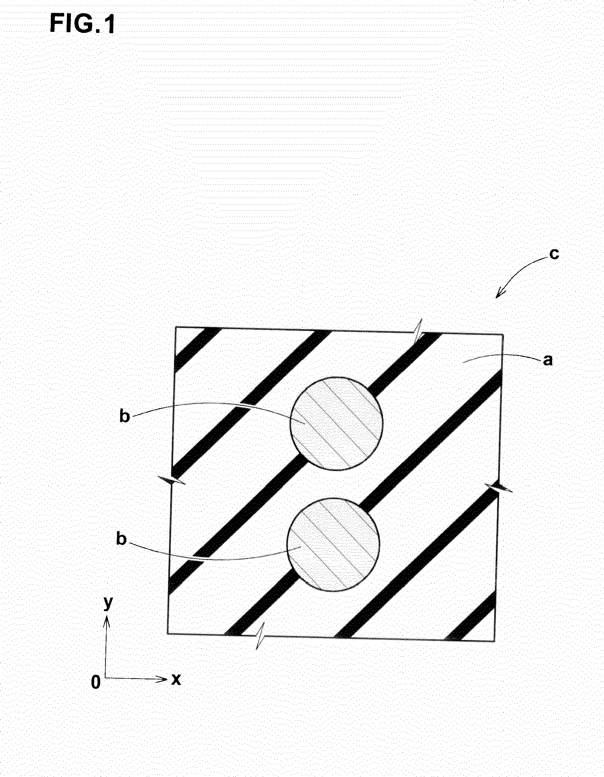

In the present invention, the analysis object to be simulated and analyzed is a rubber compound (c) comprising a rubber component (a) as the matrix rubber and filler particles (b) dispersed in the matrix rubber as shown in Fig. 1. The rubber component (a) can be, for example, natural rubber (NR), isoprene rubber (IR), butyl rubber (IIR), butadiene rubber (BR), styrene butadiene rubber (SBR), styrene isoprene butadiene rubber (SIBR), ethylene-propylene-diene rubber (EPDM), chloroprene rubber (CR), acrylonitrile butadiene rubber (NBR) and the like. The filler (b) can be carbon black, silica, clay, talc, magnesium carbonate, magnesium hydroxide and the like. Of course the rubber component (a) and filler (b) are not limited to these examples. Further, various additives, e.g. sulfur, vulcanization accelerator and the like may be added in the rubber compound (c),

In this embodiment, a slice of the rubber compound having a constant thickness (t) is used as the above-mentioned specimen 5.

* Method for simulating deformation of rubber compound

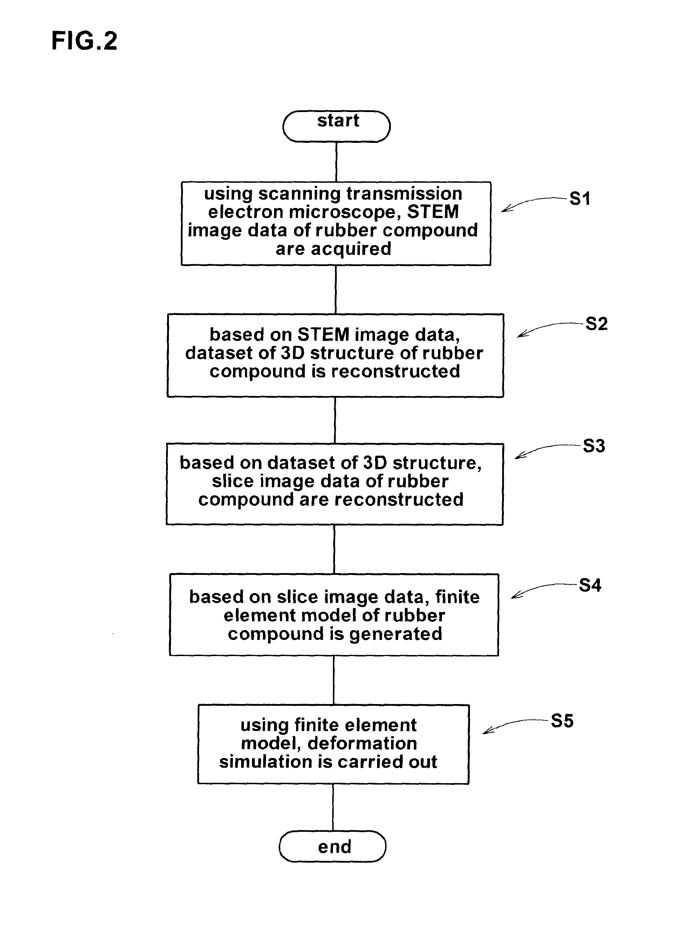

A flow chart implementing the simulating method as an embodiment of the present invention is shown in Fig. 2. This method comprises the following steps S1-S5.

** STEM image acquiring step S1

In this step S1, by the use of the scanning transmission electron microscope (STEM) 100, STEM images of the rubber compound (c) are acquired.

Incidentally, the specimen holder 6 with the specimen 5 is attached to the specimen stage 9 by an operating personnel. The electron beam 2 emitted from the electron gun 1 and accelerated by an accelerator (not shown) and focused by the focusing lens 3 is scanned on the specimen 5 by the X-direction and Y-direction scanning coils 4X and 4Y. The electrons 7, which penetrate through the specimen 5 with or without scattered, go out from the lower surface of the specimen 5. The outgoing electrons 7 travel through the holes 8 and 10 to the scattering angle limiting aperture 11 which allows the electrons having particular scattering angles to pass through it. The electrons 12 passing through the scattering angle limiting aperture 11 go into the scintillator 13, and thereby the energy of the incident electrons 12 is reemitted in the form of light. Then by the accompanying photoelectron multiplier tube 14, the light is converted to an electronic signal. The electrical signal is amplified and converted to digital data by an amplifier and A/D converter (not shown).

The digital data are transmitted to a display (not shown) in which, according to the transmitted signal, brightness modulation is made, and an electron beam transmission image reflecting the internal structure of the specimen 5 is displayed as a STEM image, and at the same time, the digital data are stored in a memory of the computer.

Thus, a plurality of STEM images corresponding to the scan positions are acquired as the STEM images' dataset.

The intensity and scattering angle of the outgoing electrons 7 are varied depending on the internal state, thickness and/or atomic species of the specimen 5. The scattering angle is also varied by the accelerating voltage. For example, if the accelerating voltage is decreased, the electrons are scattered more in the specimen 5, and the scattering angle or outgoing angle from the lower surface of the specimen 5 with respect to the central axis (O) is increased.

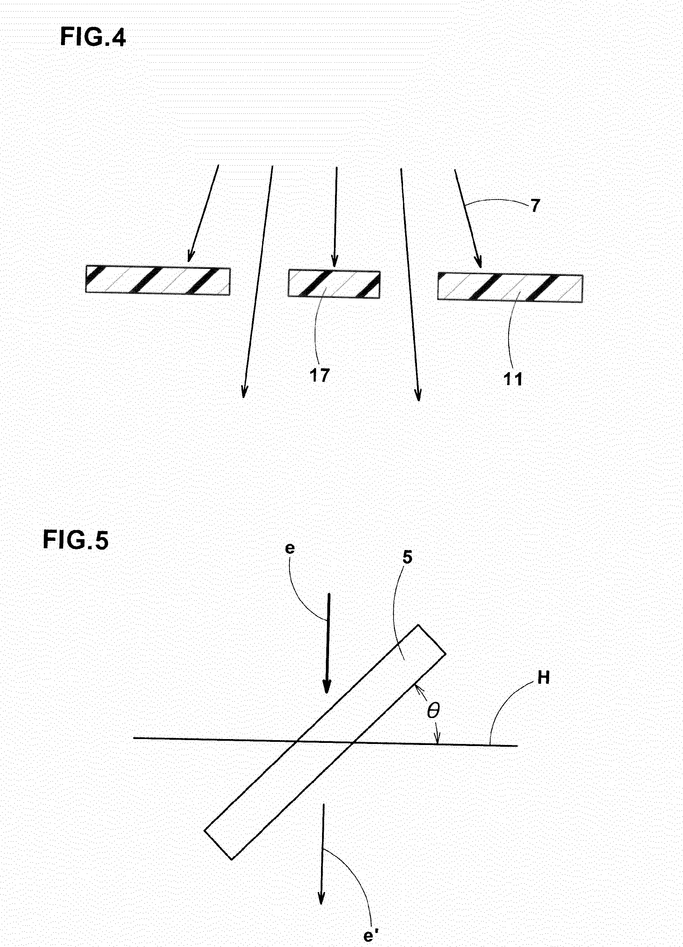

As shown in Fig. 4, the scattering angle limiting aperture 11 may be provided in its center with a masking shield 17 for further limiting the passing of the electrons 7 although the example shown in Fig. 3 is not provided with such masking shield. In general, the electron beam transmission image becomes a bright-field image when the additional masking shield is not used, but it becomes a dark-field image if the masking shield is used.

In order to create a clear image, the camera length L1 namely the distance between the specimen 5 and the scintillator 13 is preferably set in a range of from 8 to 150 cm. The accelerating voltage for the electron beam may be set in a range of 100 to 3000 kV depending on the specimen 5.

In the STEM image acquiring step S1 in this embodiment, a plurality of images of the rubber compound (c) are took from different angles with respect to the central axis (O) of the scanning transmission electron microscope 100.

For this purpose, the microscope 100 is provided with a specimen tilting device (not shown) to tilt the specimen 5 with respect to the central axis (O).

With this, as shown in Fig. 5, the specimen 5 can be held at different tilt angles $\theta$ with respect to a horizontal plane H. In this embodiment, the computer outputs a control signal to the specimen tilting device and according thereto the device tilts the specimen 5 at a specific angle $\theta$.

The variable range of the angle $\theta$ of the specimen 5 is -90 to +90 degrees, preferably -70 to +70 degrees. However, if the specimen is a round bar of the rubber compound, the variable range of the angle $\theta$ may be -180 to +180 degrees.

Firstly, the specimen 5 is tilted at a measuring start angle $\theta$ and in this tilted state, the STEM images or the dataset thereof are acquired as explained above.

Then, until a measuring stop angle θ, the process of changing the tilt angle of the specimen 5 and acquiring the dataset of the STEM images of the specimen 5 at that tilt angle are repeated at a step in a range of from 0.5 to 4 degrees, preferably 1 to 2 degrees in order to obtain the after-mentioned slice images clearly and efficiently.

Thereby, the dataset of the STEM images of the specimen inclined at different tilt angles are obtained. Incidentally, the measuring start angle θ and measuring stop angle θ can be arbitrarily set on the microscope by using a controller. In this embodiment, the measuring start angle θ is +70 degrees, and the measuring stop angle θ is -70 degrees.

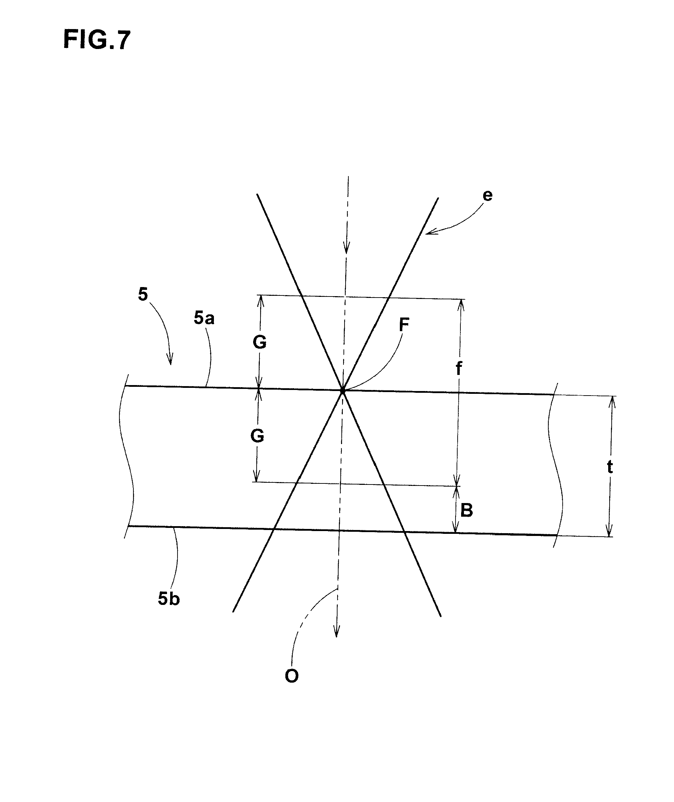

Conventionally, the focal point F of the electron beam (e) is set at the upper surface 5a of the specimen 5. In this case, there is a possibility that a clear image cannot be obtained in the vicinity of the lower surface 5b of the specimen 5. For instance, as shown in Fig. 7, if the thickness (t) of the specimen 5 is 1000 nm, the depth (f) of field of the microscope is 1200 nm (or +/-600 nm), and the focal point F is set at the surface 5a of the specimen 5, then a lower part B having 400 nm thickness of the specimen 5 is outside the depth of field, and accordingly, a clear image of such part B can not be obtained. This problem is liable to occur with increase in the thickness (t).

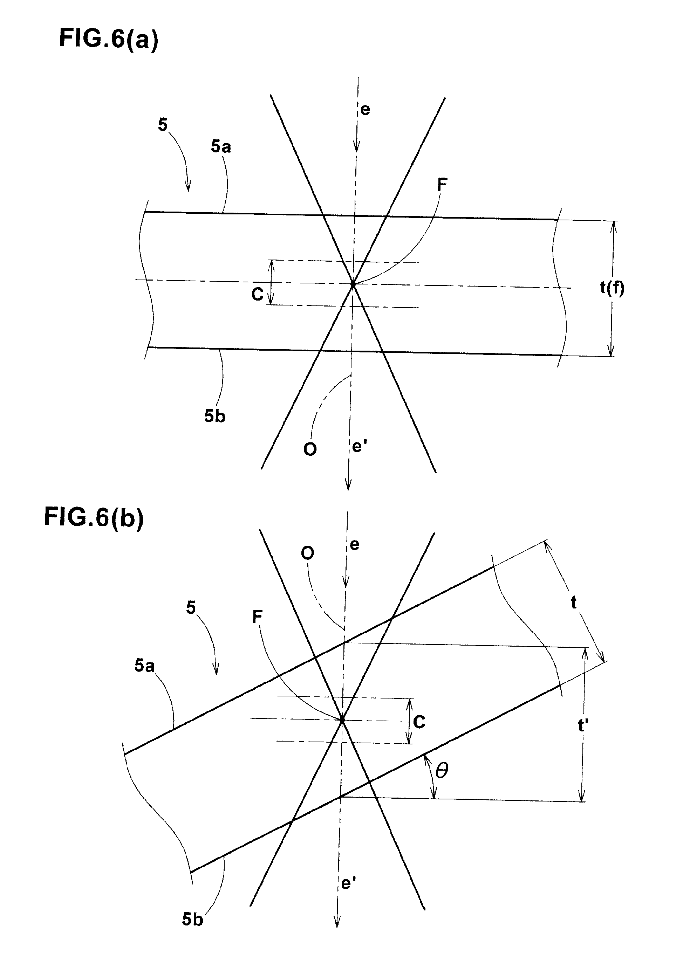

In this embodiment, therefore, the focal point F of the electron beam (e) is set in a thickness center region C of the specimen 5 as shown in Fig. 6(a). Thereby, the range on the specimen 5 in which a clear image can be obtained becomes increased. It is desirable that the depth (f) of field can completely overlaps or encompass the thickness (t) of the specimen 5.

In Fig. 6(a), the upper surface 5a and the lower surface 5b of the specimen 5 are perpendicular to the electron beam axis (namely, the incidence angle is equal to 90 deg.). In Fig. 6(b), the upper surface 5a and the lower surface 5b of the specimen 5 are inclined with respect to the electron beam axis (namely, the incidence angle is not equal to 90 deg.). Under such inclined state, the thickness of the specimen 5 measured along the electron beam axis is referred to as apparent thickness (t') in contrast to the real thickness (t) measured perpendicularly to the upper surface 5a.

From the real thickness (t) and the incidence angle, the apparent thickness can be obtained as follows

```
apparent thickness (t') =

    real thickness (t)/sin (incidence angle).
```

It is desirable that the central region C within which the focal point F is set, ranges 30 %, preferably 20 %, more preferably 10 % of the real/apparent thickness. The central region C may be off-centered, but preferably it is centered on the center of the real/apparent thickness.

The real thickness (t) may be less than 200 nm as usual, but it is preferably set in a range of from 200 to 1500 nm, more preferably 500 to 1000 nm. By increasing the thickness (t) near to 1500 nm, the dispersion of the filler particles including a compact cluster having a diameter of 200 nm or more can be accurately simulated.

Incidentally, the focal point F is adjusted by the focusing lens 3 and/or specimen stage 9 by the use of a focal point adjuster of the microscope system 100.

** Three-dimensional structure reconstruction step S2

In this step S2, from the dataset of the STEM images acquired in the step S1, a three-dimensional structure of the rubber compound is reconstructed as numerical data (hereinafter the "3D dataset") by executing a tomographic method with the computer, and the 3D dataset is stored in a memory of the computer.

As the dataset of the STEM images, those acquired by changing the tilt angle of the specimen 5 as explained above can be preferably used. But, it is also possible to use those acquired by not changing the tilt angle, namely acquired at a single tilt angle of the specimen 5 preferably zero degree with respect to the central axis (O) of the microscope 100.

From the 3D dataset, the computer can create and output various images as visual information as well as numerical data.

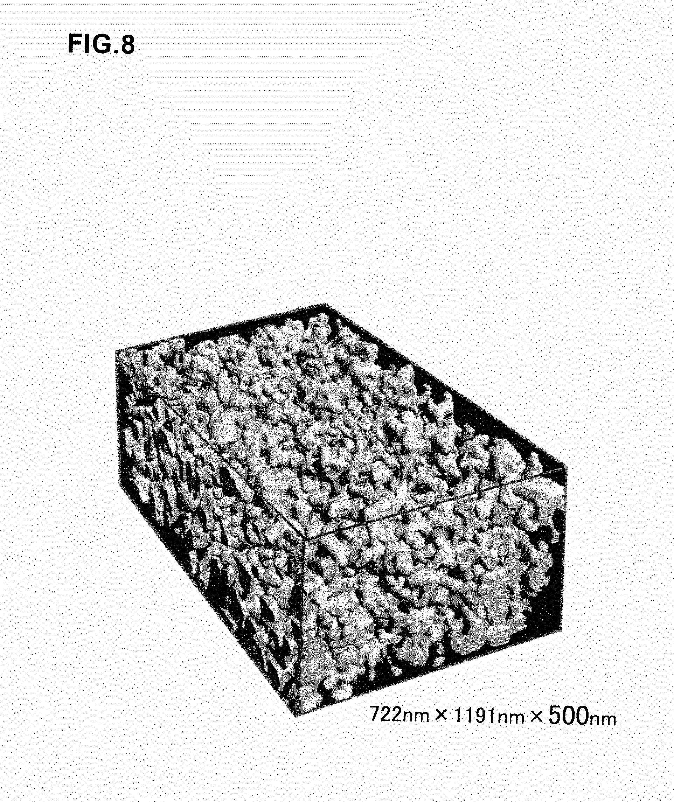

Fig. 8 shows such created image which is a perspective view of the filler particles dispersed in the rubber compound.

** Slice image acquiring step S3

In this step S3, from the reconstructed 3D dataset of the three-dimensional structure of the rubber compound, slice images of the rubber compound (c) taken along predetermined sections of the rubber compound (c) are reconstructed by the computer as numerical data (herein after the "slice image dataset"), and the slice image dataset is stored in a memory of the computer.

The above-mentioned predetermined sections of the rubber compound can be arbitrarily determined according to the coordinate system (cartesian or polar or cylindrical) employed in the subsequent step S4.

** Finite element model generating step S4

In this step S4, the slice image is subjected to an image processing to divide the entire region of the slice image into a domain of the rubber component (a), a domain of the filler particle (b) and/or a domain of other component if any.

As to the image processing method, a known method can be used in which, based on threshold levels of gray level, micro regions of the slice image are each identified whether it is a rubber domain or a filler domain (or other domain if any).

Based on the divided domains of one or more slice images, a finite element model 5a of the rubber compound is generated.

Thus, the generated model 5a of the rubber compound comprises a domain 21 of the rubber component (a), a plurality of domains 22 of the filler particles (b), and a domain of other component if any.

In the filler particle domain 22, the filler particle (b) is as usual discretized into a finite number of elements eb. In the rubber component domain 21, the rubber component (a) is as usual discretized into a finite number of elements eb.

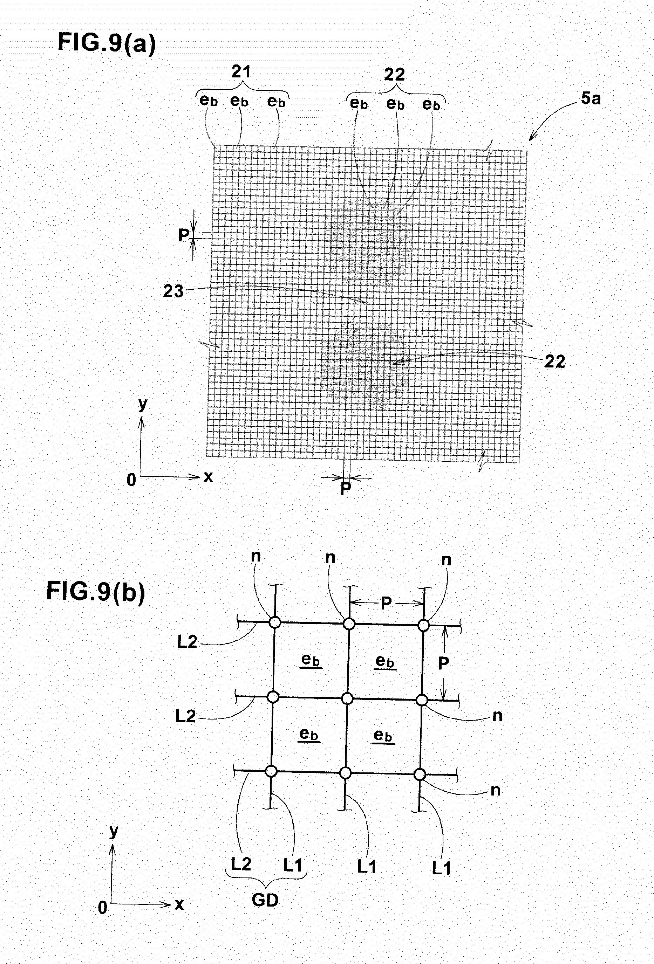

Fig. 9(a) shows a section of a small part of the rubber compound model 5a taken along a corresponding slice image, wherein the shadowed areas indicate the filler particle domains 22. Fig. 9(b) is a closeup thereof showing some elements. In this example, as shown, the rubber compound model 5a is a structured grid model having boundary GD (L1 and L2) at even intervals P in the x-axis direction, y-axis direction and z-axis direction (not shown).

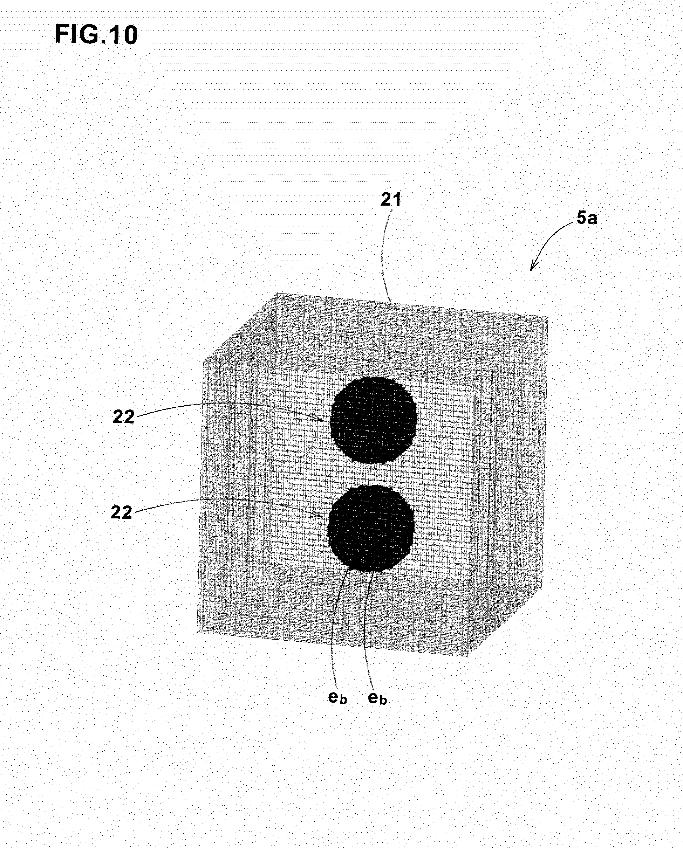

Using a plurality of slice images, a three-dimensional finite element model 5a can be generated as shown in Fig. 10. using one slice image, a two-dimensional finite element model 5a can be generated as shown in Fig. 9(a).

In the meshing or grid generation process, for example, with the computer, a grid (e.g. structured grid) is defined and superimposed onto the image-processed slice image or images, and for each element (e.g. quadrilateral element, hexahedral element or the like) of the grid, it is computed which one of the rubber component domain, filler particle domain and other domain if any has the highest proportion of area or volume in the concerned element, and the element is defined as being one having the highest proportion. Namely, the computer determines whether the element belongs to the rubber component or the filler particle or other component if any.

By employing a structured grid model, it is possible to generate the rubber compound model 5a rapidly. Further, since the grid generation is based on the slice image or images accurately created from the 3D dataset of the rubber compound, a precise model of the rubber compound can be obtained.

In this step S4, information is defined on the elements eb, which is required for simulations or numerical analyses conducted by the use of a numerical analysis method, e.g. a finite element method or the like. Such information includes at least indexes and coordinate values of node points (n) of each element eb.

Further, on each element eb, material characteristics (materials properties) of the part of the rubber compound which part is represented by the concerned element are defined. Specifically, on each of the elements eb of the rubber component domain 21 and filler particle domains 22, material constants corresponding to physical properties of the rubber component or filler are defined and stored in a memory of the computer as numerical data.

** Stress dependence defining step

In this step, in order to accurately simulate deformation of the rubber compound, the stress dependence on strain rate of the rubber component is defined on the elements of the rubber component domain 21. In order to define, a technique to change parameters in the molecular chain network theory as disclosed in Japanese patent No. 4594043 can be employed. This step can be implemented as a step separate from the former step S4, but preferably incorporated in the step S4.

** Simulation step S5

In this step S5, using the finite element model 5a of the rubber compound, a simulation of deformation of the rubber compound is carried out under given conditions. For this purpose, a known method, e.g. homogeneization method (asymptotic expansion homogeneization method) or the like can be employed.

**Comparison Tests**

In order to confirm the advantage of the method according to the invention, comparison tests were conducted.

Firstly, an explanation is given about the STEM image acquiring step S1 and three-dimensional structure reconstruction step S2. Equipments and materials used are as follows.
Scanning transmission electron microscope: JEOL Ltd. JEM-2100F Microtome: Leica Ultramicrotome EM UC6

[ Rubber materials ]

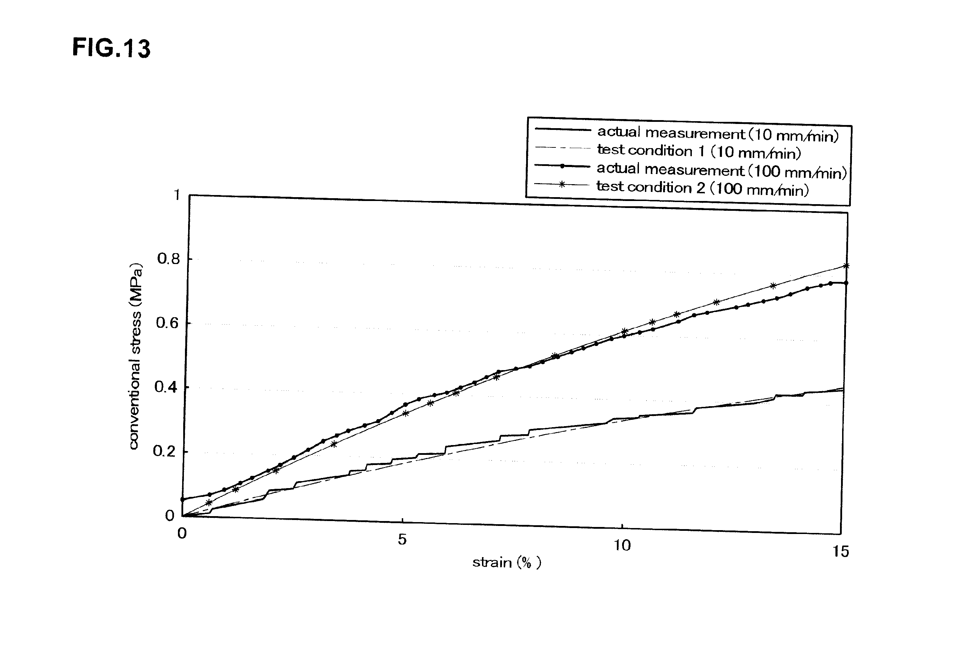

100 parts by mass of SBR (Sumitomo Chemical company, Limited: SBR1502)
53.2 parts by mass of silica (Rhodia Japan Ltd.: 115Gr)
4.4 parts by mass of silane coupling agent (Si69)
0.5 parts by mass of sulfur (Tsurumi chemical. Co. Ltd.: Powdered sulfur)
1 parts by mass of vulcanization accelerator A (Ouchi Shinko Chemical Industrial Co., Ltd.: NOCCELER NS)
1 parts by mass of vulcanization accelerator B (Ouchi Shinko Chemical Industrial Co., Ltd.: NOCCELER D)
using a banbury mixer, the materials except for the sulfur and vulcanization accelerators were kneaded for four minutes at 160 degrees C. Then, the kneaded materials to which the sulfur and vulcanization accelerators were added was further kneaded by the use of a open roll kneader for two minutes at 100 degrees C, and a raw rubber compound was prepared. The raw rubber compound was vulcanized for thirty minutes at 175 degrees C.
The vulcanized rubber was sliced by using the ultramicrotome, and a specimen having a thickness of 500 nm was prepared.
Using the STEM mode (camera length L1= 150 cm, accelerating voltage = 200 kV) of the microscope JEM-2100F, STEM images of the specimen were acquired by changing the tilt angle of the specimen from -60 to +60 degrees at a step of 1 degree, wherein, in the case of test condition 1, the focal point was set at the thickness center of the specimen, and in the case of test condition 2, the focal point was set at the upper surface of the specimen.
From the data of the STEM images obtained under each test condition, a 3D dataset of a three-dimensional structure of the rubber compound was reconstructed.

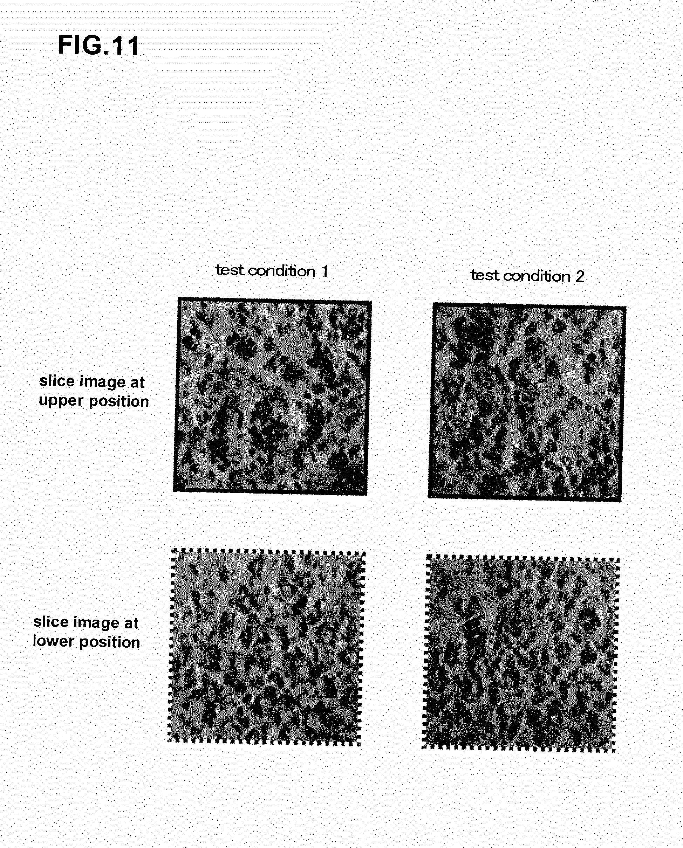

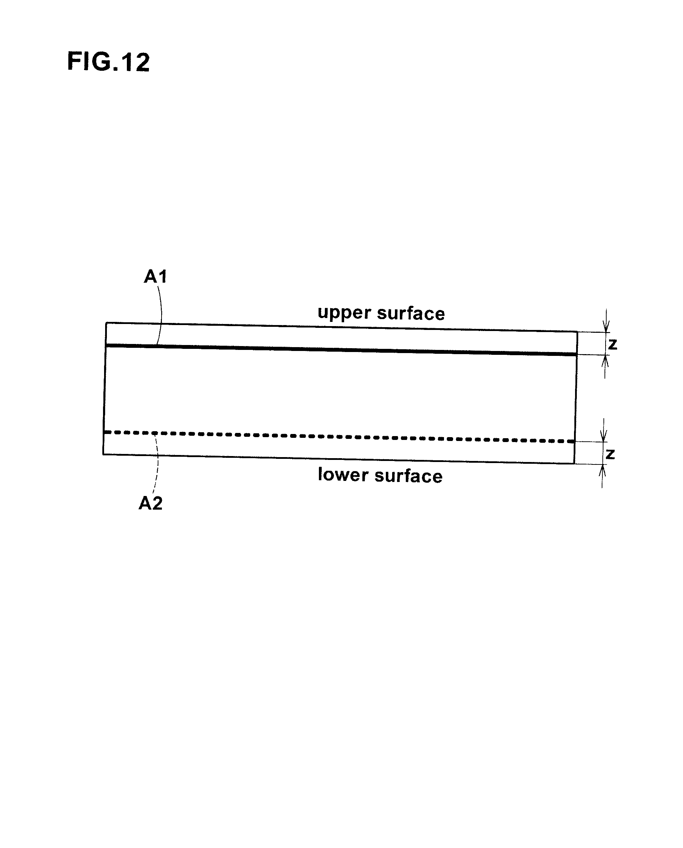

Fig. 11 shows slice images at the upper position A1 and lower position A2 of each of three-dimensional structures created from the 3D dataset. The upper and lower positions A1 and A2 are at 40 nm from the upper and lower surfaces, respectively, as shown in Fig. 12.
As shown, in the test condition 2, the image at the lower position became unclear. However, in the test condition 1, the image at the lower position as well as the image at the upper position became clear.
The above-mentioned perspective view shown in Fig. 8 was created from the 3D dataset obtained under the test condition 1.
Based on the slice image at the lower position A2 obtained in the test condition 1, a two-dimensional rubber compound model made up of square elements and having a stress dependence on strain rate were prepared. More specifically, according to the method disclosed in Japanese patent application publication JP-P2006-138810A (corresponding to US7292966, EP1657657, CN177669), different parameters for the strain rates of 10 mm/min and 100 mm/min were defined in the constitutive equation of the rubber component in the rubber compound model.
Then, using this rubber compound model, a simulation for tensile deformation was carried out to obtain the stress-strain relationship under the following conditions.
Maximum tensile strain: 3 mm
Tensile strain rate: 10 mm/min and 100 mm/min
Macroscopic region; 20 mm x 20 mm
The simulation results are shown in Fig. 13 together with the actual measurements obtained from the rubber compound. As shown, the simulation results have a high correlation with the actual measurements.

**Claims**

1. A method for simulating deformation of rubber compound including a rubber component and filler particles, comprising:

   a STEM image acquiring step in which, by the use of a scanning transmission electron microscope (STEM), data of STEM images of the rubber compound are acquired;
   a three-dimensional structure reconstruction step in which, based on the data of the STEM images, a dataset of a three-dimensional structure of the rubber compound is reconstructed;
   a finite element model generating step in which, based on the dataset of the three-dimensional structure of the rubber compound, a finite element model of the rubber compound is generated, so that the finite element model comprises a domain of the rubber component divided into a finite number of elements, and domains of the filler particles each divided into a finite number of elements;
   a stress dependence defining step in which the stress dependence on strain rate of the rubber component is defined on the elements of the rubber component; and

a simulation step in which, based on the finite element model, a simulation of deformation of the rubber compound is carried out.

2. The method according to claim 1, wherein

in the STEM image acquiring step, the focal point of the scanning transmission electron microscope is set in a thickness center region of a specimen of the rubber compound.

3. The method according to claim 1, wherein

in the STEM image acquiring step, a specimen of the rubber compound is tilted with respect to the central axis of the scanning transmission electron microscope, and

the STEM images are took at different tilt angles of the specimen of the rubber compound while the focal point of the scanning transmission electron microscope is set in a thickness center region of the specimen of the rubber compound based on an apparent thickness measured along the direction of the electron beam axis across the specimen of the rubber compound.

4. The method according to claim 1 or 2, wherein

the thickness of the specimen of the rubber compound is 200 to 1500 nm.

5. The method according to claim 1 or 2, wherein

the thickness of the specimen of the rubber compound is 200 to 1500 nm, and

the distance between the specimen of the rubber compound and a detector for the transmission electrons of the scanning transmission electron microscope is 8 to 150 cm.

# FIG.1

## FIG.2

```
         ╭─────────╮
         │  start  │
         ╰────┬────╯
              │
  ┌───────────────────────┐
  │ using scanning         │        ⟵── S1
  │ transmission           │
  │ electron microscope,   │
  │ STEM image data of     │
  │ rubber compound        │
  │ are acquired           │
  └───────────┬───────────┘
              │
  ┌───────────────────────┐
  │ based on STEM          │        ⟵── S2
  │ image data, dataset    │
  │ of 3D structure of     │
  │ rubber compound        │
  │ is reconstructed       │
  └───────────┬───────────┘
              │
  ┌───────────────────────┐
  │ based on dataset of    │        ⟵── S3
  │ 3D structure, slice    │
  │ image data of rubber   │
  │ compound are           │
  │ reconstructed          │
  └───────────┬───────────┘
              │
  ┌───────────────────────┐
  │ based on slice image   │        ⟵── S4
  │ data, finite element   │
  │ model of rubber        │
  │ compound is generated  │
  └───────────┬───────────┘
              │
  ┌───────────────────────┐
  │ using finite element   │        ⟵── S5
  │ model, deformation     │
  │ simulation is carried  │
  │ out                    │
  └───────────┬───────────┘
              │
         ╭────┴────╮
         │   end   │
         ╰─────────╯
```

## FIG.3

# FIG.4

# FIG.5

**FIG.6(a)**

**FIG.6(b)**

# FIG.7

**FIG.8**

722nm × 1191nm × 500nm

## FIG.9(a)

## FIG.9(b)

# FIG.10

# FIG.11

test condition 1    test condition 2

slice image at
upper position

slice image at
lower position

# FIG.12

A1

upper surface

z

lower surface

z

A2

FIG.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4594043 B
- JP P2006138810 A
- US 7292966 B
- EP 1657657 A
- CN 177669